# EUROPEAN PATENT APPLICATION

(11) **EP 1 251 179 A2**
(43) Date of publication of application: **23.10.2002**
(21) Application number: 02008551.0
(22) Date of filing: 16.04.2002
(51) Int. Cl.: C12N 15/68

(54) **Method for protein expression starting from stabilized linear short DNA in cell-free in vitro transcription/translation systems with exonuclease-containing lysates or in a cellular system containing exonucleases**

(30) Priority: 18.04.2001 DE 10119005
(71) Applicant: Roche Diagnostics GmbH, 68305 Mannheim (DE); F. HOFFMANN-LA ROCHE AG, 4002 Basel (CH)
(72) Inventor: Watzele, Manfred, Dr., 82362 Weilheim (DE); Hoffmann, Thomas, 68535 Neu-Edingen (DE); Nemetz, Cordula, Dr., 82515 Wolfratshausen (DE); Heindl, Dieter, Dr., 82327 Tutzing (DE); Metzler, Thomas, Dr., 80337 München (DE); Mutter, Wolfgang, Dr., 82347 Bernried (DE)

(57) **Abstract**

The present invention concerns a method for protein expression comprising the steps of transcribing stabilized linear short DNA in cell-free *in vitro* transcription/ translation systems with exonuclease-containing lysates or in a cellular system containing exonucleases and subsequent translation, wherein the stability of the linear short DNA is improved by one or several of the following measures to protect the double-stranded DNA from exonucleases:
a) incorporation of exonuclease resistant nucleotide analogues or other molecules at the 3' end of the template,
b) use of PCR primer pairs which contain exonuclease-resistant nucleotides to produce a linear short DNA,
c) protection of a template produced by a PCR reaction by connecting the 5' primer to the 3' end of the complementary strand,
d) protecting the template by DNA sequence-specific binding molecules which bind to both ends of the linear template,
e) inactivation of the exonucleases by adding competitive or non-competitive inhibitors,
f) circularization of the template to form a ring-shaped closed template.

SEQUENCE LISTING

<110>  Roche Diagnostics GmbH

<120>  Method for protein expression starting from stabilized linear short
       DNA in cell-free in vitro transcription/translation systems with
       exonuclease-containing lysates or in a cellular system containing
       exonucleases

<130>  19292

<160>  11

<170>  PatentIn version 3.1

<210>  1

<211>  20

<212>  DNA

<213>  Artificial primer

<400>  1
cgcacgcgtt ttcgcgtgcg                                                    20

<210>  2

<211>  22

<212>  DNA

<213>  Artificial primer

<400>  2
ttcgcacgcg aaaacgcgtg cg                                                 22

<210>  3

<211>  22

<212>  DNA

<213>  Artificial primer

<400>  3
aacgcacgcg aaaacgcgtg cg                                                 22

<210>  4

<211>  22

<212>  DNA

<213>  Artificial primer

<400>  4
ttgcgtgcgc ttttgcgcac gc                                                22

<210>  5

<211>  66

<212>  DNA

<213>  Artificial primer

<400>  5
gcttagatcg agatctcgat cccgcgaaat taatacgact cactataggg agaccacaac      60

ggtttc                                                                  66

<210>  6

<211>  49

<212>  DNA

<213>  Artificial primer

<400>  6
ggaagctttc agcaaaaaac ccctcaagac ccgtttagag gccccaagg                   49

<210>  7

<211>  39

<212>  DNA

<213>  Artificial primer

<400>  7
agcgcacgcg ttttcgcgtg ccgtccggcg tagaggatc                              39

<210>  8

<211>  60

<212>  DNA

<213>  Artificial primer

<400>  8
agcgcacgcg ttttcgcgtg cgcgtccggc gtagaggatc gcgatcctct acgccggacg      60

<210>  9

<211>  43

<212>  DNA

<213>  Artificial primer


<400>  9
accgctcccg gttttccggg agcggatcat ggcgaccaca ccc                    43


<210>  10

<211>  23

<212>  DNA

<213>  Artificial primer


<400>  10
tagaggatcg agatctcgat ccc                                          23


<210>  11

<211>  23

<212>  DNA

<213>  Artificial primer


<400>  11
tggcgaccac acccgtcctg tgg                                          23

## Description

The present invention concerns a method for protein expression comprising the steps of transcribing stabilized linear short DNA in cell-free *in vitro* transcription/ translation systems with exonuclease-containing lysates or in a cellular system containing exonucleases and subsequent translation, wherein the stability of the linear short DNA is improved by one or several of the following measures to protect the double-stranded DNA from exonucleases:
a) incorporation of exonuclease resistant nucleotide analogues or other exonuclease-resistant molecules at the 3' end of the template,
b) use of PCR primer pairs which contain exonuclease-resistant nucleotides to produce a linear short DNA,
c) protection of a template produced by a PCR reaction by connecting the 5' end to the 3' end of the complementary strand,
d) protecting the template by DNA sequence-specific binding molecules which bind to both ends of the linear template,
e) inactivation of the exonucleases by adding competitive or non-competitive inhibitors,
f) circularization of the template to form a ring-shaped closed template.

Cell-free DNA-dependent *in vitro* transcription/translation works quite well in practice with respect to the expression of circular double helix DNA and with respect to the expression of long linear DNA. Attempts at expressing short linear DNA pieces had only limited success. The smaller the DNA that is used the more difficult it is to obtain appreciable amounts of gene product. It was established that these difficulties were due to the presence of exonucleases. Hence it was shown that exonuclease V is responsible for the degradation of linear DNA when S30 lysates of E. coli were transcribed and translated *in vitro.* Exonuclease V is composed of three subunits (the gene products recB, recC, redD). This exonuclease cleaves the linear DNA starting at its 3' end.

It was attempted to remedy this problem by mutating the subunits of this exonuclease in order to remove the lytic activity. Yang et al., (1980) PNAS vol. 77, No. 12, pp 7029-7033 describe an improved protein synthesis starting from linear DNA templates using the E. coli strain CF300 after deletion of exonuclease V (elimination of the genes recB, recC; strain recB21).

Leavel Basset et al, J Bacteriol. (1983), vol. 156 No. 3 , pp 1359-1362, additionally mutated the RNase and polynucleotide phosphorylase genes (rna-19 pnp-7) in the recB21 strain (strain CLB7) and achieved a significantly higher protein expression with linear DNA templates after a one hour incubation period.

Lesley et al, J. Biol. Chem (1991), vol. 266 No. 4 , pp. 2632-38, used an exonuclease V-deficient recD BL21 strain which was referred to as the SL119 strain and described for the first time the method of *in vitro* protein synthesis starting from PCR-generated templates. Lysates of the strain SL119 are commercially available (Promega) for *in vitro* transcription/translation using linear templates.

However, a disadvantage of the measures described above is that all these mutants grow more slowly and also the lysates obtained from these strains have a significantly poorer rate of synthesis. Apparently this exonuclease plays an important role in the metabolism of the bacteria. Hence it appears to be important to use lysates or cell cultures in which exonucleases are present.

Single-stranded nucleic acids were protected against exonucleolytic degradation by modifying the nucleic acids either by protecting both ends or by using modified nucleotide building blocks as described in the literature for nucleic acids in the anti-sense field and in the following citations.

Single-stranded DNA/RNA molecules can be protected by protecting the ends with alkyl groups and by modifying the bases; Pandolfi et al., (1999) Nucleosides & Nucleotides. 18(9), 2051-2069. Verheijen et al. (2000) Bioorganic & Medicinal Chemistry Letters 10, 801-804 show an increased stability of single-stranded DNA molecules by protecting the ends with 4-hydroxy-N-acetylprolinol, L-serinol or by 3'-3'-phosphodiester bonds. Pure or mixed phosphorothioate bonds and chemically modified oligonucleotides e.g. methylphosphonates and phosphoramidates are more stable and are degraded more slowly by exonucleases, Kandimalla et al., NAR (1997) vol. 25. No. 2, pp 370-378. Tohda et al., (1994) Journal of Biotechnology 34 (1994) 61-69 show that RNA containing phosphorothioates is more stable towards nucleases and therefore has a higher translation efficiency. However, on the whole only small amounts of protein could be produced. Tang et al., (1993) NAR, vol. 21, No. 11, pp 2279-2735 show that hairpin loop structures protect the 3' end of single-stranded DNA's against exonucleolytic degradation. Hirao et al., (1993) FEBS, vol. 321, No. 2, 3, 169-172 show that the hairpin, which the DNA fragment d(GCGAAGC) forms, is extremely resistant to nucleases from E. coli extracts. Yoshizawa et al., (1994) NAR, Vol. 22, No. 12, pp 2217-2221 describe that a stabilization of the 3' end of mRNA by hybridization with the same hairpin results in a 200-fold increase in the efficiency of *in vitro* translation with E. coli extracts. Good and Nielsen (1998) PNAS USA 95, 2073-2076 show that synthetic molecules containing bases that are coupled to a peptide backbone (peptide nucleic acid, PNA) are resistant to hydrolytic cleavage in E. coli extracts and can be used as anti-sense molecules. Burdick and Emlen (1985) J. Immunology 135, 2593-2597 describe that in DNA anti-DNA immunocomplexes, IgG molecules can protect the DNA that is bound to them from nucleolytic degradation.

EP 0 967 274 describes methods for preparing dumbbell-shaped linear double-stranded DNA molecules. In this method a plasmid is cleaved with restriction enzymes and the resulting double-stranded non-covalently closed molecules are then modified to form dumbbell-shaped constructs by digesting the ends with a restriction endonuclease that forms single-stranded overhangs and subsequently ligating matching hairpin oligomers onto the resulting single-strand overhangs. This construct has an increased stability towards the exonucleases of T7 DNA polymerase.

Other cell-free expression systems without protection strategies are described in the prior art: In US 5571690 Hecht describes a method for the cell-free synthesis of a protein starting with a template which was generated in a PCR reaction. In this method the entire gene sequence including the phage promoter region from a plasmid is amplified. After the *in vitro* transcription a lysate from rabbit reticulocytes for the translation is used. With this method it was possible to produce 57 µg/ml of a protein using mRNA which was modified after transcription with a 5'CAP. Martemyanov et al., (1997) FEBS Lett. 414, 268-270 use an S30 extract from E, coli for the cell-free synthesis of a protein starting with a template which was generated in a 2-step PCR reaction. In this method the target gene is firstly amplified in a PCR reaction with the aid of two gene-specific oligonucleotide primers and subsequently subjected to a second PCR reaction in which a so-called megaprimer is used to fuse the T7 promoter and the ribosomal binding site to the amplified gene. It was only possible to produce radioactively detectable amounts of protein. Yang et al., (2000) J. Bacteriol. 182, 295-302 use an S30 extract from E. coli to demonstrate the cell-free synthesis of a protein starting with a template which was generated in a PCR reaction. It was only possible in this method to produce radioactively detectable amounts of protein. Nakano et al., (1999) Biotechnol. & Bioeng. 64, 194-199 use an S30 extract from E. coli in a hollow fibre reactor to at least produce 80 µg protein per ml reaction mixture starting with a template which was generated in a PCR reaction. In US 6027913 Sommer used an extract from reticulocytes for the cell-free synthesis of a protein starting with a template which was generated in a single step PCR reaction. In this method the T7 promoter and the ribosomal binding site are fused to the target gene. Even with this method only small amounts of protein were produced.

However, the methods described above are not satisfactory. Although eukaryotic lysates from rabbit reticulocytes are relatively nuclease-free, a disadvantage is that these lysates cannot be produced economically in large amounts. They only allow very small protein yields. The same applies to lysates from wheat germs which either have to be very laboriously prepared or they are otherwise strongly contaminated with translation-inhibiting factors from the surrounding tissue (JP 236 896/2000).

In contrast E. coli lysates yield much larger amounts of protein. However, the described methods for preparing lysates from E. coli only allow relatively short reaction periods of up to about one hour with linear DNA templates since afterwards these DNA templates are completely degraded by the exonuclease contained in the lysate. The lysates obtained from E. coli exonuclease mutants (i.e. exonuclease-deficient strains) have a significantly poorer synthesis performance than comparable wildtype strains such as the A19 strain for example.

The methods for protecting mRNA have the disadvantage that firstly an *in vitro* transcription has to be carried out before the protected mRNA can be added to the lysate. This in turn does not permit a coupled reaction and a continuous RNA synthesis. Methods for protecting RNA are described in Tohda et al. (1994) Journal of Biotechnology 34 (1994) 61-69, Yoshizawa et al., (1994) NAR, vol. 22, pp 2217-2221.

Hence the prior art has never provided a method which protects a double-stranded DNA against exonucleases by a modification or treatment with suitable reagents in order to use it to express protein in a cell-free lysate containing exonuclease or in cellular systems containing exonucleases. Hence the object is in particular to develop a method for protecting double-stranded DNA which, despite the protective measures to protect the DNA, enables protein expression and does not inhibit or interfere with the protein expression.

Therefore the object of the present invention was a method in which a double-stranded DNA is protected against exonucleases by a modification or treatment with suitable reagents in order to use it for protein expression in cell-free DNA-dependent *in vitro* transcription/translation systems with exonuclease-containing lysates or in cellular systems containing exonucleases.

This object is achieved according to the invention by a method for protein expression comprising the steps of transcribing stabilized linear short DNA in cell-free *in vitro* transcription/translation systems with exonuclease-containing lysates or in a cellular system containing exonucleases and subsequent translation,
characterized in that the stability of the linear short DNA is improved by one or several of the following measures to protect the double-stranded DNA from exonucleases:
a) incorporation of exonuclease resistant nucleotide analogues or other exonuclease-resistant molecules at the 3' end of the template,
b) use of PCR primer pairs which contain exonuclease-resistant nucleotides to prepare a linear short DNA,
c) protection of a template by connecting the 5' end to the 3' end of the complementary strand,
d) protecting the template by DNA sequence-specific binding molecules which bind to both ends of the linear template,
e) inactivation of the exonucleases by adding competitive or non-competitive inhibitors,
f) circularization of the template to form a ring-shaped closed template.

Exonuclease-resistant nucleotide analogues can be incorporated at the 3' end of the template according to measure a) by incorporating dideoxy-nucleoside tri-phosphates. Alternatively it is for example possible to incorporate 5' phosphothioate-protected nucleoside triphosphates or deoxy-nucleoside triphosphates. However, when terminal transferase is used for the enzymatic incorporation, it is also possible to incorporate other molecules such as para-nitrophenyl phosphate which would also be resistant to exonucleases. These molecules can also be incorporated by means of a chemical reaction.

When using PCR primer pairs which contain exonuclease-resistant nucleotides according to measure b), it is possible to incorporate 5' phosphothioate-protected nucleoside triphosphates or deoxy-nucleoside triphosphates. It would also be conceivable to use all analogues of nucleoside bases of phosphates and of deoxy riboses that can be incorporated during a chemical oligonucleotide synthesis and which, after incorporation into an oligonucleotide, can serve as a primer for one of the thermostable DNA polymerases known to a person skilled in the art in a subsequent PCR reaction.

The 5' end can be joined to the 3' end of the complementary strand according to measure c) by ligating two hairpin-forming oligonucleotide adapters to the free ends of the template. Oligonucleotide adapters in the sense of the present invention are for example SEQ ID NO. 1: 5'-PO₄-C GCA CGC GTT TTC GCG TGC G-OH-3'. The oligonucleotide adapters are for example ligated by using T4 DNA ligase.

The 5' end can be joined to the 3' end of the complementary strand according to measure c) by replacing one or several nucleoside monomer units in the primer sequence by one or several deoxyribose phosphates or corresponding abasic analogues such as (1-phospho-(3,4)hydroxybutanediol) during the chemical synthesis of the primers and using these PCR primers in the PCR. These deoxyribose phosphates or abasic analogues lead to the termination of the polymerase reaction and thus to a single-stranded DNA end at the 5' end of the template. This free 5' end forms a hairpin-shaped loop with itself and its 5' end can for example be ligated to the 3' end of the complementary strand using T4 DNA ligase. A similar method is also conceivable in which nucleotide analogues instead of the abasic linkers are incorporated into the primers whose bases or riboses are for example modified by silyl groups and thus prevent their extension by the polymerase.

The 5' primer can be joined to the 3' end of the complementary strand according to measure c) by incorporating a chemical crosslinker such as psoralen at the 5' end of the two PCR primers and creating the chemical bond after the PCR reaction by means of for example a reaction under the action of strong light as in the case of psoralen.

The 5' end can also be joined to the 3' end of the complementary strand according to measure c) by incorporating one or several nucleotides or nucleotide analogues such as uridine into the PCR primer which are removed again by a subsequent chemical reaction with bases or an enzymatic reaction with uracil N-glycosylase after the PCR to form a 3' overhang and this 3' overhang is then constructed according to the invention in such a manner that it forms a hairpin-shaped loop with itself and its 3' end lies exactly opposite to the 5' end of the complementary strand and the DNA gap is closed by subsequent ligation for example with T4 DNA ligase resulting in a dumbbell-shaped internal ring closure. The following oligonucleotide can for example be used for this as the sense primer (x₁-xₙ are nucleotides which are homologous to the target sequence to be amplified):

SEQ ID NO: 2
5'-ttc gca cgc gaa aac gcg tgc g-P-uridine-P-uridine-P-x1-xn-3'.

If a base or uracil-N-glycosylase is used for the cleavage and the double-strand is melted by heating, the 5' primer drops off up to the nucleotides x₁-xₙ (2.) and the 3' end formed by the previous PCR can then hybridize with itself.
1. 
SEQ ID NO: 3
SEQ ID NO: 4
5'-aac gca cgc gaa aac gcg tgc g-P-uridine-P-uridine-P-x1-xn-3'
3'-ttg cgt gcg ctt ttg cgc acg c-P-adenine-P-adenine-P- -y1-yn-5'
2. 
SEQ ID NO: 4
5'-P-x1-xn-3'
3'-ttg cgt gcg ctt ttg cgc acg c-P-adenine-P-adenine-P- -y1-yn-5'
3.

SEQ ID NO: 4

t
/ \
t c gcg tgc g-P-thymine-P-thymine 5'P-x1-xn-3'
t g cgc acg c-P-adenine-P-adenine- P -y1-yn-5'
\ /
t

The 5' primer can also be connected to the 3' end of the complementary strand according to measure c) by incorporating a molecule at the 5' end of both PCR primers and also incorporating a nucleotide modified with this molecule or another molecule at the 3' end of the template and joining these two molecules at the 3' end and at the 5' end in a non-covalent manner by means of a protein. For example the two molecules can be biotin and the protein can be avidin or streptavidin. In this case biotin is incorporated at the 5' end via the chemically synthesized oligonucleotide as a biotinylated nucleotide and is introduced at the 3' end as a biotinylated nucleotide triphosphate using terminal transferase. Since biotin has a high affinity to avidin or streptavidin, and avidin or streptavidin can bind up to 4 molecules of biotin, avidin or streptavidin can connect the two opposing biotin residues of the two strands. The two molecules can also be digoxigenin and the protein can be an antibody directed against digoxigenin or the digoxigenin binding part of an antibody. In this case the procedure is similar to that using avidin/streptavidin and biotin.

According to measure d) it is conceivable that the DNA sequence-specific binding molecule which binds to both ends of the linear template is an antibody directed against this DNA sequence or the DNA binding part of an antibody.
According to the invention the DNA sequence-specific binding molecule of measure d) which binds to the two ends of the linear template can be a PNA molecule or several PNA molecules which hybridize to the 3' and/or 5' ends of the final template.

The protection afforded by measure d) is basically that large molecules bind to the two ends of the linear template. Hence biotinylated ends to which streptavidin/avidin binds would therefore for example also be conceivable or digoxigenylated ends to which a dig antibody or dig-binding part of the antibody binds.

The exonucleases can be inactivated according to measure e) by adding unspecific DNA which competitively inhibits the activity of the exonucleases.

The exonucleases can be inactivated according to measure e) by adding inactivating antibodies which block the activity of the exonucleases.

The present invention also concerns the method of the invention according to measure f) in which both ends of the linear template are biotinylated for example using terminal transferase and streptavidin or avidin is used to connect both ends to form a ring. According to measure f) it is also possible to digoxigenylate both ends of the linear template and to bind an antibody directed against digoxigenin or the digoxigenin binding part of an antibody to connect both ends.
The present invention also concerns the method of the invention according to measure f) in which the two ends of the linear template are circularized via a DNA molecule and a subsequent enzymatic, chemical or non-covalent ligation. A direct ligation of the two ends e.g. with T4 DNA ligase would be thermodynamically unfavourable and would only proceed very inefficiently and hence most of it would remain unligated and thus susceptible to exonuclease attack. It is therefore necessary to generate very long overhangs at the ends of the DNA which when paired with a complementary DNA piece are converted into a thermodynamically preferred state and thus allow an efficient ligation.

For this purpose a template is prepared by a PCR reaction in which for example such primers are used which lead to a termination of the PCR reaction and thus to 5' overhangs. Suitable primers in this regard are in particular those having introduced one or several modified monomer units in the primer sequence which prevent extension of a template by a polymerase. Modified monomer units according to the invention are for example deoxyribose phosphates, abasic analogues or in particular nucleotide analogues whose base or ribose is modified and thus prevents extension by the polymerase. Alternatively primers can also be used for the PCR which can be subsequently wholly or partially removed again analogous to claim 10. This PCR product which now has either 5' or 3' overhangs at its ends can be circularized by ligation with another DNA piece when this has overhangs that are complementary to the template. These DNA pieces can be chemically synthesized or they can be also provided with the appropriate overhangs by the same PCR method. The latter method is particularly preferred when the ligation with a DNA molecule occurs by generating overhanging 5' ends or 3' ends in the template and in the DNA molecule which are complementary to one another. The overhanging 3' ends are generated by incorporating one or several nucleotides or nucleotide analogues which can be removed again by subsequent chemical or enzymatic reaction after the PCR reaction to form a 3' overhang. The overhanging 5' ends are generated by incorporating one or several modified monomer units which prevent the extension of a template by a polymerase. A PCR reaction is subsequently carried out. Termination of the polymerase reaction at the modified sites results in a free single-stranded DNA end at the 5' end of the template. The modified monomer units can also be deoxyribose phosphates or abasic analogues. The modified monomer units can also be nucleotide analogues whose base or ribose is modified and which thus prevent the extension by the polymerase.

One prerequisite for *in vitro* protein synthesis is the production of a DNA template. This template must contain the following elements: A promoter for the RNA polymerase that is used, a ribosomal binding site and the target gene to be expressed. In principle it is possible to use a linear or a circular closed template. There are various methods for generating a linear template for example by linearizing a plasmid with the aid of restriction endonucleases. A linear template can also be produced very simply by means of the PCR method. Whereas it is very simple to carry out an *in vitro* transcription with a purified RNA polymerase using linear templates, linear DNA templates are susceptible to exonuclease attack in a coupled *in vitro* transcription-translation mixture: Since the ribosomes, aminoacyl tRNA synthases, initiation, elongation and termination factors required for the translation can only be prepared from lysates as a mixture together with exonucleases, it is necessary to protect linear templates against exonucleolytic attack.

This can be achieved by modifying the ends of the template or by inactivating the exonucleases by adding one ore more competitive or non-competitive inhibitors. However, in this connection it is important that neither the transcription nor the individual steps of the translation are inhibited. Thus for example EDTA is known to be an effective agent against a number of nucleases but EDTA at the same time also interferes with transcription as well as translation.

It is now possible for the RNA polymerase which is either present in the lysate or is added separately, to transcribe the mRNA from such a protected DNA template. This mRNA is now translated on the ribosomes. In order for the measures stated in the claims not to interfere with the transcription, care must be taken that the modifications at the ends are not inserted directly in the promoter region but in front of the promoter. When using avidin or streptavidin it is important to use an excess of avidin or streptavidin over the biotin residues on the DNA to prevent aggregation of the entire DNA. In addition the remaining free binding sites on the avidin or streptavidin must be saturated with biotin before the template is used in the reaction. Otherwise a complete inhibition of the protein synthesis would surprisingly occur. When using antibodies care must be taken that they do not react unspecifically with DNA or other essential proteins from the lysate.

### Figures

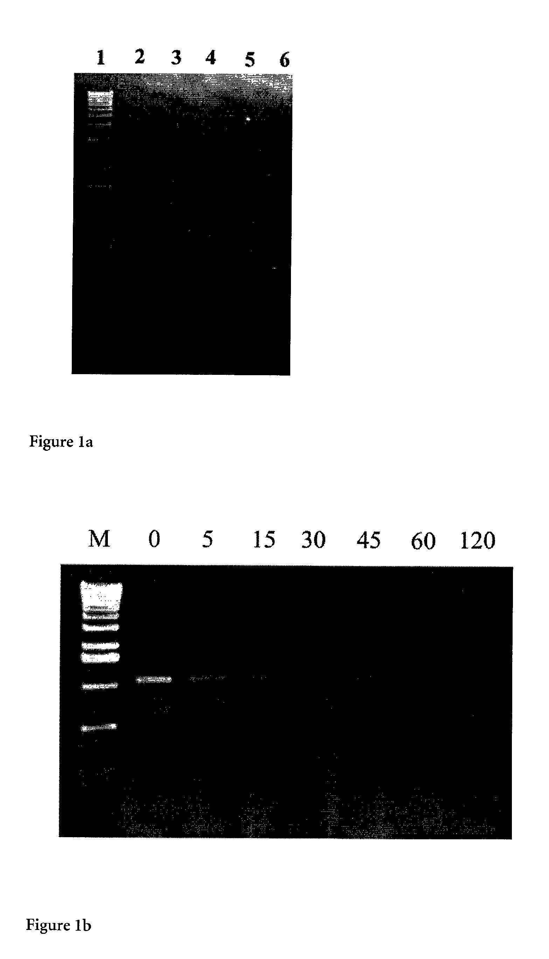

Figure 1a
   shows the stability of an unmodified template. Already after 5 minutes linear DNA is no longer detectable in this mixture.
   lane 1 standard,
   lanes 2-6 linear DNA after 5, 15, 30, 45 and 60 minutes incubation in the *in vitro* transcription-translation mixture.
Figure 1b
   shows the stability of a template whose 3' ends were modified with dideoxy-ATP using terminal transferase. Even after 10 minutes linear DNA is still detectable in this mixture.
Figure 2
   shows an improvement of the protein synthesis yield by modifying the 3' ends of the linear DNA template with dideoxy-ATP or with phosphothioate-ATP in comparison with non-modified templates. As indicated 1 µg and 2 µg DNA were used.
Figure 3a
   shows the stability of a template generated according to example 2 with overhanging 5' ends before ligation. In contrast to the unmodified template of figure 1a, it is only after 10 minutes that linear DNA can no longer be detected in this case.
Figure 3b
   shows the stability of a template generated according to example 2with overhanging 5' ends after ligation. After ligation the template is present for the entire synthesis period of 120 minutes. The degradation of the linear DNA was greatly reduced.
Figure 4
   shows an improvement of the protein synthesis yield by modifying the 3' ends of the linear DNA template according to example 2. The flipping over of the 5' ends already resulted in a higher synthesis of the protein. However, it is not until the two ends are ligated that the higher stability of the template is achieved and the largest amount of protein is formed. As stated 1 µg and 2 µg DNA were used.
Figure 5
   shows the stability of the template towards exonuclease III. Whereas the unmodified DNA is already degraded by 10 U exonuclease III, the DNA ligated to psoralen is also stable towards 100 U exonuclease III.
   lanes 1-3 psoralen ligated DNA, lanes 4-6 unmodified DNA. Lane 1,4 without exonuclease III, lane 2,5 with 10 U exonuclease III, lane 3,6 with 100 U exonuclease III.
Figure 6
   shows an improvement of the protein synthesis yield by the psoralen linkage of the 3' ends of the linear DNA template.
Figure 7
   shows an improvement of the protein synthesis yield by modifying the primers with biotin and subsequent conjugation with streptavidin.

### Example 1:

Protection against 3' exonucleases by incorporating non-hydrolysable nucleotides at the 3' end of the template with terminal transferase. In this case dideoxy nucleotide triphosphates or phosphothioate-ATP were incorporated. The degradation of the template was greatly reduced by these modifications and the protein expression yield was increased several-fold.

### PCR reaction

A 1115 bp fragment was amplified with the Expand High Fidelity PCR kit (Roche Diagnostics GmbH) for the *in vitro* expression starting with PCR fragments. 50 ng pIVEX2.1 GFP was used as the template. The plasmid pIVEX2.1 GFP contains the sequence for the green fluorescent protein from Aequorea victoria in the form of a mutant GFPcycle 3 (Nature Biotechnology (1996) 14, 315-319) with the following important control elements for *in vitro* expression: T7 promoter, ribosomal binding site and T7 terminator. The PCR product began 30 bp upstream of the T7 promoter and contained the GFP-coding sequence up to the end of the T7 terminator. The following primers were used for the amplification:

Sense primer
SEQ ID NO: 5
5' gcttagatcgagatctcgatcccgcgaaattaatacgactcactatagggagaccacaacggtttc and
antisense primer
SEQ ID NO: 6
5'ggaagctttcagcaaaaaacccctcaagacccgtttagaggccccaagg.

The PCR cycle of 1 min 94°C, 1 minute 60°C, 1 minute 72°C was repeated 30 times. The concentration of the product was estimated by means of an agarose gel. The PCR product was then precipitated with ethanol and taken up in DNAse and RNAse-free water.

### Modification of the 3' ends with dideoxy-ATP with the aid of terminal transferase

45 µg PCR product was incubated for 40 min at 37°C with 250 U terminal transferase (Roche Diagnostics GmbH) and 30 nmol dideoxy-ATP (Roche Diagnostics GmbH) in 100 µl 5 x reaction buffer for terminal transferase (Roche Diagnostics GmbH) containing 2.5 mM CoCl₂ in a total volume of 500 µl and then precipitated with ethanol and taken up in 20 µl DNAse and RNAse-free water.

### Modification of the 3' ends with phosphothioate-ATP with the aid of terminal transferase

10 µg PCR product was incubated for 40 min at 37°C with 75 U terminal transferase (Roche Diagnostics GmbH) and 47 nmol phosphothioate-ATP (adenosine 5'-O-(1-thiotriphosphate) NAPS Company, Göttingen, Germany #39565 N) with 10 µl 5 x reaction buffer for terminal transferase (Roche Diagnostics GmbH) containing 2.5 mM CoCl₂ in a total volume of 50 µl and then precipitated with ethanol and taken up in 10 µl DNAse and RNAse-free water.

### Coupled in vitro transcription/translation reaction

Transcription/translation reactions were carried out in a volume of 50 µl for 2 hours at 30°C. The reaction solution contained 80.5 mM potassium acetate, 10 mM magnesium acetate, 35 mM ammonium chloride, 4 mM magnesium chloride, 4 % polyethylene glycol 2000, 1 mM ATP, 0.5 mM CTP, 1 mM GTP, 0.5 mM UTP, 30 mM phosphoenolpyruvate, 8 µg/ml pyruvate kinase, 400 µM of each amino acid (all 20 naturally occurring amino acids), 0,1 mM folic acid, 0,1 mM EDTA, 50 mM HEPES-KOH pH 7.6/30°C, 20 µg/ml rifampicin, 0.03 % sodium azide, 2 µg/ml aprotinin, 1 mg/ml leupeptin, 1 µg/ml pepstatin A, 10 mM acetylphosphate, 100 µg/ml tRNA from E. coli MRE600, 8 mM dithiothreitol, 100 U/ml Rnase-inhibitor, 15 µl E. coli lysate, 0.5 U/µl T7-RNA polymerase. The E. coli lysate was prepared from the A19 strain by the method of Zubay (Annu. Rev. Genet. (1973) 7, 267). If not stated otherwise 1 µg DNA template which was prepared by the methods described in the respective examples, was added to each mixture.

### Exonuclease assay

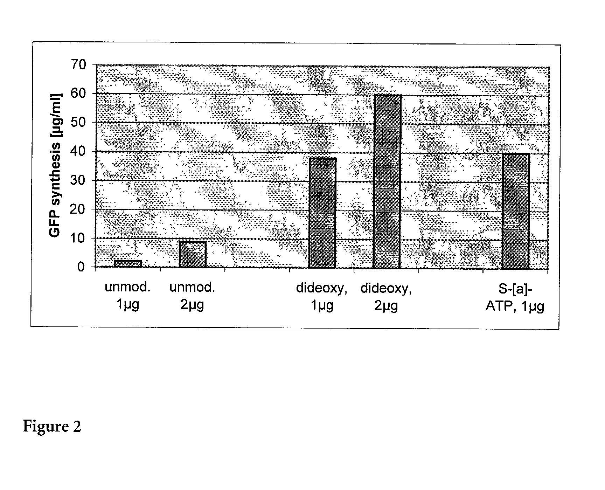

13 µl sample was taken at the stated times in minutes from a coupled *in vitro* transcription/translation reaction (200 µl total reaction) and heated for 15 min at 65°C. After cooling on ice for 15 min, 107 µl H₂O and 3 µl RNAse (Roche #119915) were added and incubated for 30 min at 37°C. Then 12 µl 5 % SDS and 3 µl proteinase K (Roche #1413783) were added and incubated for 30 min at 37°C. It was subsequently precipitated with 13 µl 3 M NaAc (pH 4.8) and 400 µl ice-cold ETOH for 30 min at -20°C and, after washing with 200 µl ice-cold 70 % ETOH and drying, the entire amount was applied to a 1 % TBE gel (see figures 1a, 1b and 2).

### Example 2:

### Protection from 5' and 3' exonucleases by connecting the 5' primer to the 3' end of the complementary strand

PCR primers in which two nucleoside monomer units in the sequence are replaced by two abasic linkers (in this case simply deoxyriboses) were used in a PCR. As described in EP 0 416 817 these sites lead to the termination of the polymerase reaction and thus to a single-stranded DNA end at the 5' end of the template. This free 5' end was constructed such that it formed a hairpin-shaped loop with itself and lay exactly opposite to the 3' end of the complementary strand. The DNA gaps were closed by subsequent ligation to form a dumbbell-shaped internal ring closure.
See sketch

### Sketch

### oligonucleotide:

SEQ ID NO: 7
5'-agc gca cgc gtt ttc gcg tgc g5'ribose3'-5'ribose3'-P-cgt ccg gcg tag agg
atc g-3'

### PCR product with an overhang at the 3' end

SEQ ID NO: 8
5'-agcgcacgcgttttcgcgtgcg-ribose-ribose-cgtccggcgtagaggatcg
                                        3'-gcaggccgcatctcctagc

### After the overhang has flipped over

SEQ ID NO: 8
ttcgcgtgcg-P-ribose-P-ribose-P-cgtccggcgtagaggatcg...
ttgcgcacgc - g - a gcaggccgcatctcctagc...
5' 3'

### After ligation

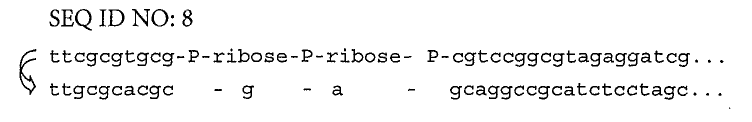
SEQ ID NO: 8

 ttcgcgtgcg-P-ribose-P-ribose- P-cgtccggcgtagaggatcg...
ttgcgcacgc    - g    - a    -    gcaggccgcatctcctagc...

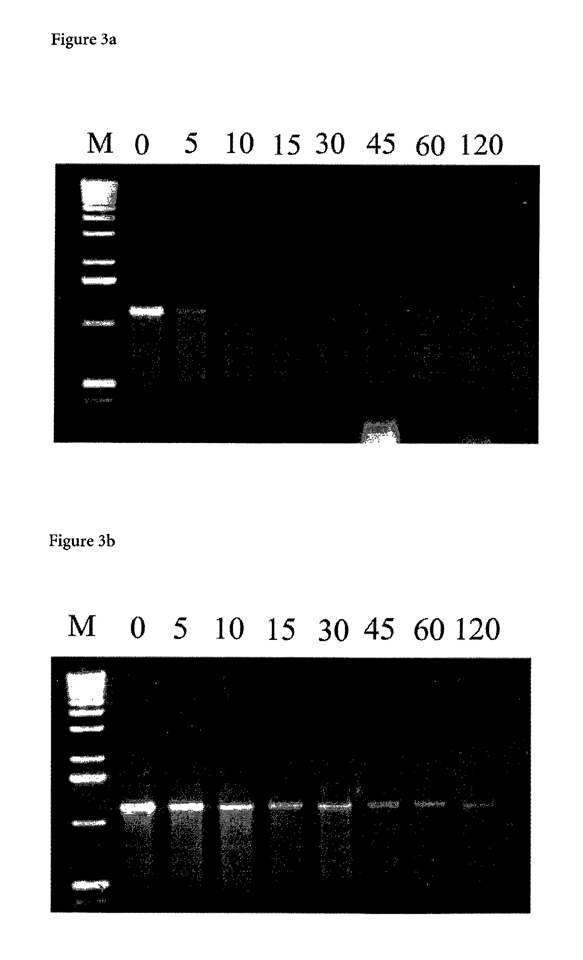

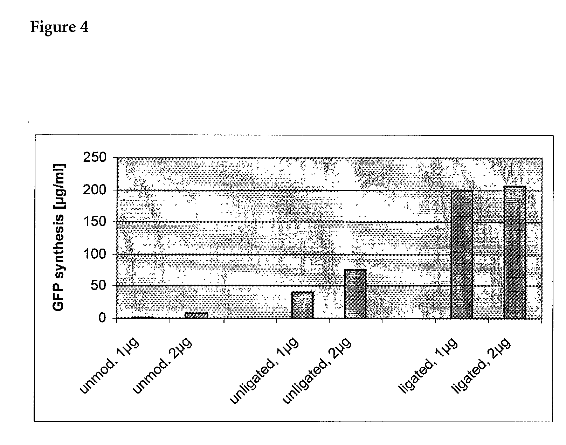

It was possible to greatly reduce the degradation of the template by these modifications and the protein expression yield was increased several-fold (see figures 3a, 3b and 4).

### PCR reaction

For *in vitro* expression a 1115 bp fragment was amplified using the Expand High Fidelity PCR kit (Roche Diagnostics GmbH) starting from PCR fragments. The PCR product began 25 bp upstream of the T7 promoter and contained the GFP-coding sequence up to the end of the T7 terminator. The following primers were used for the amplification (ribose denotes a β-2'-deoxy-D-ribofuranose; P denotes a phosphate group).

Sense primer
SEQ ID NO: 7
5'-agc gca cgc gtt ttc gcg tgc g-P-5'ribose3'-P-5'ribose3'-P-cgt ccg gcg tag
agg atc g-3'

Antisense primer
SEQ ID NO: 9
5'-acc gct ccc ggt ttt ccg gga gcg g-P-5'ribose3'-P-5'ribose3'-P-atc atg gcg
acc aca ccc gt-3'.

300 ng pIVEX2.1 GFP was used as the template. The plasmid pIVEX2.1 GFP contains the sequence for the green fluorescent protein from Aequorea victoria in the form of a mutant GFPcycle 3 (Nature Biotechnology (1996) 14, 315-319) with the following important control elements for *in vitro* expression: T7 promoter, ribosomal binding site and T7 terminator.

The PCR cycle of 1 min 94°C, 1 minute 60°C, 1 minute 72°C was repeated 24 times. The concentration of the product was estimated by means of an agarose gel. The PCR product was then precipitated with ethanol and taken up in DNAse and RNAse-free water.

### Ligation of the 5' ends to the 3' end of the complementary strand

15 µg DNA from the previous PCR reaction was ligated for 18 h at 16°C with 30 units T4 DNA ligase in 180 µl ligase buffer, subsequently precipitated with ethanol and taken up in 20 µl DNAse and RNAse-free water.

The PCR and ligation result in the hairpin-shaped closed end of the linear template shown in the previous sketch.

### Exonuclease assay

The exonuclease assay was carried out analogously to example 1.

### Example 3:

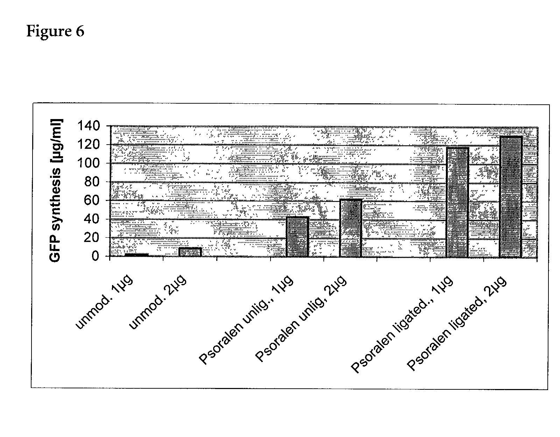

A chemical crosslinker (e.g. psoralen) was incorporated at the 5' ends of both PCR primers and after appropriate activation by light the crosslinker formed a covalent bond with the 3' end of the complementary strand via the base of the complementary strand. This resulted in a high degree of resistance to exonucleases. The template modified in this manner was used successfully to synthesize protein *in vitro* analogously to example 1 (see figure 6).

### PCR reaction

The PCR reaction was carried out as in example 1 using the following primers:

Psoralen-C-2'phosphoramidite was obtained from Glen Research Ltd., Virginia USA.

Sense primer
SEQ ID NO: 10
5'-psoralen-tagaggatcgagatctcgatccc-3'
Antisense primer
SEQ ID NO: 11
5'-psoralen-tggcgaccacacccgtcctgtgg-3'

300 ng pIVEX2.1 GFP was used as the template. The PCR cycle of 1 min 94°C, 1 minute 50°C, 1 minute 72°C was repeated 25 times. The concentration of the product was estimated by means of an agarose gel. The PCR product was then precipitated with ethanol and taken up in DNAse and RNAse-free water.

### Photochemical ligation of the 5' ends to the 3' end of the complementary strand

Psoralen was linked to the thymidine of the complementary strand by irradiating the PCR product for 5 minutes with a mercury vapour lamp in front of which there was a Pyrex filter.

### Exonuclease assay

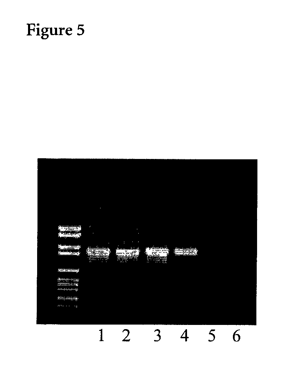

After the photochemical ligation 400 ng psoralen-modified DNA was incubated for one hour at 37°C with 10 U or 100 U exonuclease III (Roche Diagnostics GmbH) in a total volume of 20 µl (see figure 5).

### Example 4:

Protection from 5' and 3' exonucleases by using PCR primer pairs that are biotinylated at the 5' end. Streptavidin then binds to the 5' ends of the amplified template and thus protects the 5' and 3' end by its size or alternatively the streptavidin binds to the 5' ends of an amplified template and protects the two ends of the template by an internal ring closure.

A biotinylated nucleotide was incorporated at the 5' end of the two PCR primers. Then the PCR-amplified template was incubated with streptavidin whose size protects the 5' and 3' end of the template against exonucleolytic degradation. The protein was successfully synthesized from the template modified in this manner.

### PCR reaction

The PCR reaction was carried out as in example 1 using the following primers:

Sense primer
SEQ ID NO: 5
5'-biotin-gcttagatcgagatctcgatcccgcgaaattaatacgactcactatagggagaccacaacggtttc-
3'

Antisense primer
SEQ ID NO: 6
5'-biotin-ggaagctttcagcaaaaaacccctcaagacccgtttagaggccccaagg-3'

The biotinylated primers were obtained from the Metabion Company, Martinsried, Germany.

300 ng pIVEX2.1 GFP was used as the template. The PCR cycle of 1 min 94°C, 1 minute 50°C, 1 minute 72°C was repeated 25 times. The concentration of the product was estimated by means of an agarose gel. The PCR product was then precipitated with ethanol and taken up in DNAse and RNAse-free water.

### Incubation with streptavidin

10 µg streptavidin was added to 1.5 µg PCR product in a total volume of 10 µl. Subsequently the free biotin binding sites were saturated with 10 µg biotin.

### In vitro expression

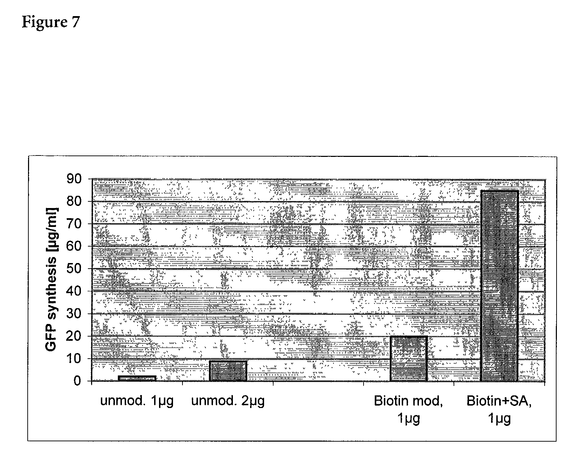

1 µg biotin/streptavidin-modified DNA was used for the *in vitro* expression as described in example 1 (see figure 7).

## Claims

1. Method for protein expression comprising the steps of transcribing stabilized linear short DNA in cell-free *in vitro* transcription/ translation systems with exonuclease-containing lysates or in a cellular system containing exonucleases and subsequent translation, wherein the stability of the linear short DNA is improved by one or several of the following measures to protect the double-stranded DNA from exonucleases:
a) incorporation of exonuclease resistant nucleotide analogues or other molecules at the 3' end of the template,
b) use of PCR primer pairs which contain exonuclease-resistant nucleotides to produce a linear short DNA,
c) protection of a template produced by a PCR reaction by connecting the 5' end to the 3' end of the complementary strand,
d) protecting the template by DNA sequence-specific binding molecules which bind to both ends of the linear template,
e) inactivation of the exonucleases by adding competitive or non-competitive inhibitors,
f) circularization of the template to form a ring-shaped closed template

2. Method as claimed in claim 1, wherein dideoxy nucleoside triphosphates are incorporated as the exonuclease-resistant nucleotide according to measure a).

3. Method as claimed in claim 1, wherein 5'-thosphothioate-protected nucleoside triphosphates or deoxy-nucleoside triphosphates are incorporated as the exonuclease-resistant nucleotide according to measure a).

4. Method as claimed in claim 1, wherein 5'-phosphothioate-protected nucleoside triphosphates or deoxy-nucleoside triphosphates are incorporated as the exonuclease-resistant nucleotide according to measure b).

5. Method as claimed in claim 1, wherein the 5' primer is connected to the 3' end of the complementary strand according to measure c) by ligating two hairpin-forming oligonucleotide adaptors to the free ends of the template.

6. Method as claimed in claim 1, wherein the 5' primer is connected to the 3' end of the complementary strand according to measure c) by
- introducing one or several modified monomer units in the primer sequence which prevent extension of a template by a polymerase,
- carrying out a PCR reaction with these PCR primers that contain one or several modified monomer units,
- termination of the polymerase reaction at the modified sites and formation of a free single-stranded DNA end at the 5' end of the template and wherein this free 5' end additionally forms a hairpin-shaped loop with itself and its 5' end is ligated to the 3' end of the complementary strand.

7. Method as claimed in claim 6, wherein the modified monomer units are deoxyribose phosphates or abasic analogues.

8. Method as claimed in claim 6, wherein the modified monomer units are nucleotide analogues whose base or ribose is modified and thus prevents extension by the polymerase.

9. Method as claimed in claim 1, wherein the 5' primer is connected to the 3' end of the complementary strand according to measure c) by incorporating a chemical crosslinker at the 5' end of the two PCR primers and carrying out the chemical linking after the PCR reaction.

10. Method as claimed in claim 1, wherein the 5' primer is connected to the 3' end of the complementary strand according to measure c) by incorporating one or several nucleotides or nucleotide analogues into the PCR primer which are removed again by a subsequent chemical or enzymatic reaction after the PCR reaction to form a 3' overhang and this 3' overhang forms a hairpin-shaped loop with itself and its 3' end lies exactly opposite to the 5' end of the complementary strand and the DNA gap is closed by subsequent ligation resulting in a dumbbell-shaped internal ring closure.

11. Method as claimed in claim 1, wherein the 5' primer can also be connected to the 3' end of the complementary strand according to measure c) by incorporating a molecule at the 5' end of both PCR primers and also incorporating a nucleotide modified with this molecule or another molecule at the 3' end of the template and joining these two molecules at the 3' end and at the 5' end in a non-covalent manner by means of a protein.

12. Method as claimed in claim 11, wherein the two molecules are biotin and the protein is avidin or streptavidin.

13. Method as claimed in claim 11, wherein the two molecules are digoxigenin and the protein is an antibody directed against digoxigenin or the digoxigenin binding part of an antibody.

14. Method as claimed in claim 1, wherein according to measure d) the DNA sequence-specific binding molecule which binds to both ends of the linear template is an antibody directed against this DNA sequence or the DNA binding part of an antibody.

15. Method as claimed in claim 1, wherein the DNA sequence-specific binding molecule of measure d) which binds to the two ends of the linear template is a PNA molecule or several PNA molecules which hybridize to the 3' and/or 5' ends of the final template.

16. Method as claimed in claim 1, wherein the exonucleases are inactivated according to measure e) by adding unspecific DNA which competitively inhibits the activity of the exonucleases.

17. Method as claimed in claim 1, wherein the exonucleases are inactivated according to measure e) by adding inactivating antibodies which block the activity of the exonucleases.

18. Method as claimed in claim 1, wherein according to measure f) the two ends of the linear template are circularized by means of a DNA molecule and a subsequent enzymatic, chemical or non-covalent ligation.

19. Method as claimed in claim 18, wherein the ligation with a DNA molecules is preferably carried out by generating overhanging 5' ends or 3' ends in the template and in the DNA molecule which are complementary to one another, wherein the method stated in claim 10 is used to produce the overhanging 3' ends and the method stated in claim 6, 7 or 8 is used to prepare the overhanging 5' ends.

20. Method as claimed in claim 1, wherein according to measure f) the ends of the linear template are biotinylated and both ends are connected by streptavidin or avidin.

21. Method as claimed in claim 1, wherein according to measure f) the ends of the linear template are digoxigenylated and an antibody directed against digoxigenin or the digoxigenin binding part of an antibody connects the ends.
